(19) Europäisches Patentamt
European Patent Office
Office européen des brevets

(11) **EP 1 641 739 B1**

(12) **EUROPÄISCHE PATENTSCHRIFT**

(45) Veröffentlichungstag und Bekanntmachung des Hinweises auf die Patenterteilung:
**09.04.2014 Patentblatt 2014/15**

(21) Anmeldenummer: **04739794.8**

(22) Anmeldetag: **11.06.2004**

(51) Int Cl.:
*C07C 67/347* (2006.01)     *C07C 69/593* (2006.01)
*C07C 67/56* (2006.01)      *C07C 67/303* (2006.01)
*C07C 51/353* (2006.01)     *C07C 51/47* (2006.01)
*C07C 57/13* (2006.01)      *C07C 51/36* (2006.01)
*C07C 55/14* (2006.01)

(86) Internationale Anmeldenummer:
**PCT/EP2004/006297**

(87) Internationale Veröffentlichungsnummer:
**WO 2004/113263 (29.12.2004 Gazette 2004/53)**

(54) **VERFAHREN ZUR KONTINUIERLICHEN HERSTELLUNG EINER VERBINDUNG, DIE MINDESTENS ZWEI FUNKTIONELLE GRUPPEN TRÄGT**

METHOD FOR THE CONTINUOUS PRODUCTION OF A COMPOUND THAT CARRIES AT LEAST TWO FUNCTIONAL GROUPS

PROCEDE DE PRODUCTION, EN CONTINU, D'UN COMPOSE COMPORTANT AU MOINS DEUX GROUPES FONCTIONNELS

(84) Benannte Vertragsstaaten:
**AT BE BG CH CY CZ DE DK EE ES FI FR GB GR HU IE IT LI LU MC NL PL PT RO SE SI SK TR**

(30) Priorität: **25.06.2003 DE 10328715**

(43) Veröffentlichungstag der Anmeldung:
**05.04.2006 Patentblatt 2006/14**

(73) Patentinhaber: **BASF SE**
**67056 Ludwigshafen (DE)**

(72) Erfinder:
• **STÜER, Wolfram**
**67056 Ludwigshafen (DE)**
• **SCHEIDEL, Jens**
**69493 Hirschberg (DE)**
• **VOSS, Hartwig**
**67227 Frankenthal (DE)**
• **BASSLER, Peter**
**68519 Viernheim (DE)**
• **RÖPER, Michael**
**67157 Wachenheim (DE)**

(56) Entgegenhaltungen:
**EP-A- 0 475 386     WO-A-96/34687**
**US-A- 4 638 084**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Erfindung betrifft ein Verfahren zur kontinuierlichen Herstellung einer Verbindung, die mindestens zwei funktionelle Gruppen, unabhängig voneinander ausgewählt aus der Gruppe bestehend aus Nitrilgruppe, Carbonsäuregruppe, Carbonsäureestergruppe, Carbonsäureamidgruppe, trägt, umfassend die Schritte

a) Addition zweier terminaler Olefine, die die zur Herstellung der mindestens zwei funktionellen Gruppen enthaltenden Verbindung gemäß a1) erforderlichen funktionellen Gruppen tragen, in Gegenwart eines als Katalysator für diese Addition geeigneten, bezüglich der Reaktionsmischung homogenen Verbindung gemäß a3) unter Erhalt einer Mischung, enthaltend

a1) eine durch einfache Addition der genannten zwei terminalen Olefine erhaltene Verbindung, die mindestens zwei funktionelle Gruppen, unabhängig voneinander ausgewählt aus der Gruppe bestehend aus Nitrilgruppe, Carbonsäuregruppe, Carbonsäureestergruppe, Carbonsäureamidgruppe trägt,
a2) eine durch mehrfache Addition der genannten zwei terminalen Olefine erhaltene Verbindung und
a3) die als Katalysator für diese Addition geeignete, bezüglich der Reaktionsmischung homogene Verbindung

b) Destillation der in Schritt a) erhaltenen Mischung unter Erhalt

b1) der durch einfache Addition der genannten zwei terminalen Olefine erhaltenen Verbindung, die mindestens zwei funktionelle Gruppen, unabhängig voneinander ausgewählt aus der Gruppe bestehend aus Nitrilgruppe, Carbonsäuregruppe, Carbonsäureestergruppe, Carbonsäureamidgruppe trägt, als Kopfprodukt und
b2) einer Mischung enthaltend
b2a) durch einfache Addition der genannten zwei terminalen Olefine erhaltene Verbindung, die mindestens zwei funktionelle Gruppen, unabhängig voneinander ausgewählt aus der Gruppe bestehend aus Nitrilgruppe, Carbonsäuregruppe, Carbonsäureestergruppe, Carbonsäureamidgruppe trägt,
b2b) eine durch mehrfache Addition der genannten zwei terminalen Olefine erhaltene Verbindung und
b2c) die als Katalysator für diese Addition geeignete, bezüglich der Reaktionsmischung homogene Verbindung,

c) Auftrennung der gesamten in Schritt b2) erhaltenen Mischung oder eines Teils davon mittels einer semipermeablen Membran unter Erhalt eines Permeats und eines Retentats derart, daß das Gewichts-Verhältnis der Komponente b2b) zur Komponente b2c) in der Schritt c) zugeführten Mischung b2) kleiner ist als im Retentat,

d) das in Schritt c) erhaltene Permeat teilweise oder vollständig in Schritt a) zurückführt
und

e) den nicht gemäß c) aufgetrennten Teil der in Schritt b2) erhaltenen Mischung teilweise oder vollständig in Schritt a) zurückführt

wobei man als terminale Olefine zwei Olefine einsetzt, die unabhängig voneinander die Formel $H_2C=CHR^1$ aufweisen, in der $R^1$ für eine Nitrilgruppe, Carbonsäuregruppe, Carbonsäureestergruppe, Carbonsäureamidgruppe, steht.
Zahlreiche Verbindungen, die zwei funktionelle Gruppen, unabhängig voneinander ausgewählt aus der Gruppe bestehend aus Nitrilgruppe, Carbonsäuregruppe, Carbonsäureestergruppe, Carbonsäureamidgruppe, tragen, haben eine große technische Bedeutung.
So stellen beispielsweise Adipinsäure oder deren Derivate wichtige Ausgangsverbindungen zur Herstellung technisch bedeutsamer Polymere, wie Polyamid 6 oder Polyamid 66, dar.
Solche Verbindungen können beispielsweise erhalten werden durch Addition zweier terminaler Olefine, die die zur Herstellung der mindestens zwei funktionelle Gruppen enthaltenden monoolefinisch ungesättigten Verbindung erforderlichen funktionellen Gruppen tragen.
So kann Hexendisäurediester durch Addititon von Acrylsäureester in Gegenwart entsprechender Katalysatorsysteme, insbesondere homogener, Rhodium enthaltender Katalysatorsysteme, hergestellt werden, wie dies beispielsweise in J. Organomet. Chem. 1987, 320, C56, US 4,451,665, FR 2,524,341, US 4,889,949, Organometallics, 1986, 5, 1752, J. Mol. Catal. 1993, 85, 149, US 4,594,447, Angew. Chem. Int. Ed. Engl., 1988, 27. 185, US 3,013,066, US, 4,638,084, EP-A-475 386, JACS 1991, 113, 2777-2779, JACS 1994, 116, 8038-8060 beschrieben ist.
Bei einer solchen Addition zweier terminaler Olefine, die die zur Herstellung der mindestens zwei funktionelle Gruppen enthaltenden monoolefinisch ungesättigten Verbindung erforderlichen funktionellen Gruppen tragen, werden monoolefinisch ungesättigte Verbindungen erhalten, die mindestens zwei funktionelle Gruppen, unabhängig voneinander ausgewählt aus der Gruppe bestehend aus Nitrilgruppe, Carbonsäuregruppe, Carbonsäureestergruppe, Carbonsäureamidgruppe tragen. Durch Hydrierung können aus solchen monoolefinisch ungesättigten Verbindungen die entsprechenden gesättigten Verbindungen erhalten werden.

Für ein technisch durchführbares und wirtschaftliches Verfahren ist es wünschenswert, die Herstellung von Verbindungen, die mindestens zwei funktionelle Gruppen, unabhängig voneinander ausgewählt aus der Gruppe bestehend aus Nitrilgruppe, Carbonsäuregruppe, Carbonsäureestergruppe, Carbonsäureamidgruppe, tragen, kontinuierlich durchführen zu können. Solche Verfahren sind aus dem Stand der Technik nicht bekannt.

Der vorliegenden Erfindung lag die Aufgabe zugrunde, ein Verfahren bereitzustellen, das die kontinuierliche Herstellung einer Verbindung, die mindestens zwei funktionelle Gruppen, unabhängig voneinander ausgewählt aus der Gruppe bestehend aus Nitrilgruppe, Carbonsäuregruppe, Carbonsäureestergruppe, Carbonsäureamidgruppe, trägt, auf technisch einfache und wirtschaftliche Weise ermöglicht.

Demgemäß wurde das eingangs definierte Verfahren gefunden.

Die im Sinne der vorliegenden Erfindung als Katalysator bezeichneten Strukturen beziehen sich auf die Verbindungen, die als Katalysator eingesetzt werden; die Strukturen der unter den jeweiligen Reaktionsbedingungen katalytisch aktiven Spezies können sich hiervon unterscheiden, werden aber von dem genannten Begriff "Katalysator" mit umfasst.

Erfindungsgemäß erhält man gemäß Schritt a) durch Addition zweier terminaler Olefine, die die zur Herstellung der mindestens zwei funktionellen Gruppen enthaltenden Verbindung gemäß a1) erforderlichen funktionellen Gruppen tragen, in Gegenwart einer als Katalysator für diese Addition geeigneten, bezüglich der Reaktionsmischung homogenen Verbindung gemäß a3) eine Mischung, enthaltend

a1) eine durch einfache Addition der genannten zwei terminalen Olefine erhaltene Verbindung, die mindestens zwei funktionelle Gruppen, unabhängig voneinander ausgewählt aus der Gruppe bestehend aus Nitrilgruppe, Carbonsäuregruppe, Carbonsäureestergruppe, Carbonsäureamidgruppe trägt,

a2) eine durch mehrfache Addition der genannten zwei terminalen Olefine erhaltene Verbindung und

a3) die als Katalysator für diese Addition geeignete, bezüglich der Reaktionsmischung homogene Verbindung.

Im Sinne der vorliegenden Erfindung wird unter einer Verbindung a1) eine einzige solche Verbindung oder ein Gemisch solcher Verbindungen verstanden.

Im Sinne der vorliegenden Erfindung wird unter einer Verbindung a2) eine einzige solche Verbindung oder ein Gemisch solcher Verbindungen verstanden.

Im Sinne der vorliegenden Erfindung wird unter einer Verbindung a3) eine einzige solche Verbindung oder ein Gemisch solcher Verbindungen verstanden.

Vorteilhaft kann man als terminale Olefine zwei gleiche oder unterschiedliche, vorzugsweise gleiche, Olefine einsetzen, die unabhängig voneinander die Formel $H_2C=CHR^1$ aufweisen, in der $R^1$ für eine Nitrilgruppe, Carbonsäuregruppe, Carbonsäureestergruppe oder Carbonsäureamidgruppe, vorzugsweise Carbonsäureestergruppe oder Nitrilgruppe, steht.

Im Falle der Carbonsäureestergruppe kommen vorteilhaft Ester von aliphatischen, aromatischen oder heteroaromatischen Alkoholen, insbesondere aliphatischen Alkoholen in Betracht. Als aliphatische Alkohole können vorzugsweise $C_1$-$C_{10}$-Alkanole, insbesondere $C_1$-$C_4$-Alkanole, wie Methanol, Ethanol, i-Propanol, n-Propanol, n-Butanol, i-Butanol, s-Butanol, t-Butanol, besonders bevorzugt Methanol eingesetzt werden.

Die Carbonsäureamidgruppen können N- oder N,N-substituiert sein, wobei die N,N-Substitution gleich oder unterschiedlich, vorzugsweise gleich sein kann. Als Substituenten können vorzugsweise aliphatische, aromatische oder heteroaromatische Substituenten in Betracht, insbesondere aliphatische Substituenten, besonders bevorzugt $C_1$-$C_4$-Alkylreste, wie Methyl, Ethyl, i-Propyl, n-Propyl, n-Butyl, i-Butyl, s-Butyl, t-Butyl, besonders bevorzugt Methyl eingesetzt werden.

In einer vorteilhaften Ausführungsform kann man als terminales Olefin mit funktioneller Gruppe Acrylsäure oder deren Ester einsetzen. Die Herstellung von Acrylsäure, beispielsweise durch Gasphasenoxidation von Propen oder Propan in Gegenwart heterogener Katalysatoren, und die Herstellung von Acrylsäureestern, beispielsweise durch Veresterung von Acrylsäure mit den entsprechenden Alkoholen in Gegenwart homogener Katalysatoren, wie p-Toluolsulfonsäure, sind an sich bekannt.

Üblicherweise werden Acrylsäure bei der Lagerung oder der Verarbeitung ein oder mehrere Stabilisatoren zugesetzt, die beispielsweise die Polymerisation oder die Zersetzung der Acrylsäure vermeiden oder reduzieren, wie p-Methoxy-Phenol oder 4-Hydroxy-2,2,4,4-tetramethyl-piperidin-N-oxid ("4-Hydroxy-TEMPO").

Solche Stabilisatoren können vor dem Einsatz der Acrylsäure oder deren Ester in dem Additionsschritt teilweise oder vollständig entfernt werden. Die Entfernung des Stabilisators kann nach an sich bekannten Verfahren, wie Destillation, Extraktion oder Kristallisation, erfolgen.

Solche Stabilisatoren können in der Acrylsäure oder deren Ester in der zuvor eingesetzten Menge verbleiben.

Solche Stabilisatoren können der Acrylsäure oder deren Ester vor der Additionsreaktion zugesetzt werden.

Setzt man unterschiedliche Olefine ein, so werden bei der Addition üblicherweise Mischungen der verschiedenen möglichen Additionsprodukte erhalten.

Setzt man ein Olefin ein, so wird bei der Addition, die in diesem Fall üblicherweise als Dimerisierung bezeichnet wird, ein Additionsprodukt erhalten. Aus wirtschaftlichen Gründen ist diese Alternative meist bevorzugt.

In einer bevorzugten Ausführungsform kommt als monoolefinisch ungesättigte Verbindung, die mindestens zwei funktionelle Gruppen, unabhängig voneinander ausgewählt aus der Gruppe bestehend aus Nitrilgruppe, Carbonsäuregruppe, Carbonsäureestergruppe, Carbonsäureamidgruppe trägt, Hexendisäurediester, insbesondere Hexendisäuredimethylester, in Betracht unter Erhalt von Adipinsäurediester, insbesondere Adipinsäuredimethylester, durch Hydrierung.

Aus Adipinsäurediester, insbesondere Adipinsäuredimethylester kann Adipinsäure durch Spaltung der Estergruppe erhalten werden. Hierzu kommen an sich bekannte Verfahren zur Spaltung von Estern in Betracht.

In einer weiteren bevorzugten Ausführungsform kommt als monoolefinisch ungesättigte Verbindung, die mindestens zwei funktionelle Gruppen, unabhängig voneinander ausgewählt aus der Gruppe bestehend aus Nitrilgruppe, Carbonsäuregruppe, Carbonsäureestergruppe, Carbonsäureamidgruppe trägt, Butendinitril in Betracht unter Erhalt von Adipodinitril durch Hydrierung.

In einer weiteren bevorzugten Ausführungsform kommt als monoolefinisch ungesättigte Verbindung, die mindestens zwei funktionelle Gruppen, unabhängig voneinander ausgewählt aus der Gruppe bestehend aus Nitrilgruppe, Carbonsäuregruppe, Carbonsäureestergruppe, Carbonsäureamidgruppe trägt, 5-Cyanopentensäureester, insbesondere 5-Cyanopentensäuremethylester, in Betracht unter Erhalt von 5-Cyanovaleriansäureester, insbesondere 5-Cyanovaleriansäuremethylester, durch Hydrierung.

Die genannte Addition zweier terminaler Olefine unter Erhalt der Mischung gemäß Schirtt a) kann nach an sich bekannten Verfahren erfolgen, wie sie beispielsweise in J. Organomet. Chem. 1987, 320, C56, US 4,451,665, FR 2,524,341, US 4,889,949, Organometallics, 1986, 5, 1752, J. Mol. Catal. 1993, 85, 149, US 4,594,447, Angew. Chem. Int. Ed. Engl., 1988, 27. 185, US 3,013,066, US, 4,638,084, EP-A-475 386, JACS 1991, 113, 2777-2779, JACS 1994, 116, 8038-8060 beschrieben sind.

Die Additionsreaktion kann teilweise oder vollständig erfolgen. Demgemäß kann bei teilweisem Umsatz die Reaktionsmischung nicht umgesetztes Olefin enthalten.

Die Additionsreaktion kann vorteilhaft in Gegenwart von Wasserstoff durchgeführt werden. Dabei hat sich ein Wasserstoffdruck im Bereich von 0,1 bis 1 MPa als vorteilhaft erwiesen.

Die Addition kann vorteilhaft in Gegenwart einer bezüglich der Reaktionsmischung homogenen Verbindung, die Rhodium, Ruthenium, Palladium oder Nickel, vorzugsweise Rhodium enthält, als Katalysator durchgeführt werden.

In einer bevorzugten Ausführungsform kann die in Schritt a) erhaltene Mischung zwischen den Schritten a) und b) hydriert werden unter Erhalt einer gesättigten Verbindung.

Die Hydrierung kann vorteilhaft in Gegenwart einer bezüglich der Reaktionsmischung heterogenen Substanz als Katalysator durchgeführt werden.

Als heterogene Katalysatoren kommen vorzugsweise solche in Betracht, die als katalytische aktive Komponente ein Edelmetall der Gruppe 8 des Periodensystems der Elemente, wie Palladium, Ruthenium, Rhodium, Iridium, Platin, Nickel, Cobalt, Kupfer, vorzugsweise Palladium, enthalten.

Diese Metalle können in ungeträgerter Form, beispielsweise als Suspensionskatalysatoren, vorzugsweise im Falle von Nickel oder Cobalt eingesetzt werden.

Diese Metalle können in geträgerter Form, beispielsweise auf Aktivkohle, Metalloxide, Übergangsmetalloxide, insbesondere Aluminiumoxid, Siliziumdioxid, vorzugsweise als Festbettkatalysatoren, eingesetzt werden.

Die Hydrierung kann vorteilhaft in Gegenwart einer bezüglich der Reaktionsmischung homogenen Verbindung, die Rhodium, Ruthenium, Palladium oder Nickel, vorzugsweise Rhodium enthält, als Katalysator durchgeführt werden.

In einer bevorzugten Ausführungsform kann Schritt a) in Gegenwart der gleichen, bezüglich der Reaktionsmischung homogenen, Rhodium enthaltenden Verbindung als Katalysator durchgeführt werden wie diese Hydrierung der gemäß Schritt a) erhaltenen Mischung.

In einer besonders bevorzugten Ausführungsform kann man diese Hydrierung der gemäß Schritt a) erhaltenen Mischung durchführen ohne eine Abtrennung oder Abreicherung der in Schritt a) eingesetzten homogenen, Rhodium enthaltenden Verbindung.

Diese Verfahrensweise stellt gegenüber dem Stand der Technik einen großen Vorteil dar, da eine Aufarbeitung des bei der genannten Additionsreaktion erhaltenen Reaktionsaustrags entfällt. In einer insbesondere bevorzugten Ausführungsform kann die in Schritt a) erhaltene Mischung ohne Aufarbeitungsschritt in diese Hydrierung überführt werden.

Dies kann beispielsweise durch Überführung der in Schritt a) erhaltenen Mischung aus der Rektionsapparatur in eine weitere, für die Hydrierung vorgesehene Apparatur erfolgen, also durch eine räumliche Trennung von Schritt a) und Hydrierung. So kann beispielsweise Schritt a) in einem Reaktor, wie einem Rührkessel, einer Rührkesselkaskade, oder einem Strömungsrohr oder in einer Kombination einer dieser Reaktorarten mit einem weiteren für die Hydrierung geeigneten Reaktor durchgeführt werden.

Dies kann beispielsweise erfolgen, indem man Schritt a) und Hydrierung nacheinander in dem gleichen Apparat durchführt, also durch eine zeitliche Trennung von Schritt a) und Hydrierung.

Vorzugsweise kann man die Addition gemäß Schritt a) oder die Hydrierung oder beides in Gegenwart einer bezüglich der Reaktionsmischung homogenen, Rhodium enthaltenden Verbindung der Formel $[L^1RHL^2L^3R]^+X^-$ als Katalysator durchführen, worin

$L^1$    ein anionischer Pentahapto-Ligand, vorzugsweise Pentamethylcyclopentadienyl, ist;

$L^2$    für einen neutralen 2-Elektronendonor steht;

$L^3$    für einen neutralen 2-Elektronendonor steht;

R    ausgewählt wird aus der Gruppe, bestehend aus H, $C_1$-$C_{10}$-Alkyl-, $C_6$-$C_{10}$-Aryl-und $C_7$-$C_{10}$-Aralkyl-Liganden

$X^-$    für ein nichtkoordinierendes Anion stehtvorzugsweise für eine solches aus der Gruppe bestehend aus $BF_4^-$, B(perfluorphenyl)$_4^-$, B(3,5-bis(trifluormethyl)-phenyl)$_4^-$, Al(OR$^F$)$_4^-$ wobei R$^F$ für gleiche oder unterschiedliche teilfluorierte oder perfluorierte aliphatische oder aromatische Reste, insbesondere für Perflour-iso-propyl oder Perfluor-tert.-butyl, steht; und

und worin zwei oder drei von $L^2$, $L^3$ und R gegebenenfalls verbunden sind.

In einer bevorzugten Ausführungsform können $L^2$ und $L^3$ unabhängig voneinander ausgewählt sein aus der Gruppe bestehend aus $C_2H_4$, $CH_2$=$CHCO_2Me$, P(OMe)$_3$ und $MeO_2C$-($C_4H_8$)-$CO_2Me$.

In einer weiteren bevorzugten Ausführungsform können $L^2$ und $L^3$ miteinander verbunden sein. In diesem Fall können $L^2$ und $L^3$ zusammen insbesondere Acrylnitril oder 5-Cyanopentensäureester darstellen.

In einer weiteren bevorzugten Ausführungsform können $L^2$ und R miteinander verbunden sein. In diesem Fall können $L^2$ und R zusammen insbesondere -$CH_2$-$CH_2CO_2Me$ darstellen.

In einer weiteren bevorzugten Ausführungsform können $L^2$, $L^3$ und R miteinander verbunden sein. In diesem Fall können $L^2$, $L^3$ und R zusammen insbesondere $MeO_2C(CH_2)_2$-(CH)-($CH_2$)$CO_2Me$ darstellen.

In einer insbesondere bevorzugten Ausführungsform kann man die Addition gemäß Schritt a) oder die Hydrierung oder beides durchführen in Gegenwart einer bezüglich der Reaktionsmischung homogenen, Rhodium enthaltenden Verbindung als Katalysator ausgewählt aus der Gruppe bestehend aus

$[Cp*Rh(C_2H_4)_2H]^+ BF_4^-$,

$[Cp*Rh(P(OMe)_3)(CH_2*CHCO_2Me)(Me)]^+ BF_4^-$,

$[Cp*Rh(-CH_2-CH_2CO_2Me)(P(OMe)_3)]^+ BF_4^-$,

$[Cp*Rh(MeO_2C(CH_2)_2-(CH-)-(CH_2)CO_2Me)]^+BF_4^-$,

$[Cp*Rh(C_2H_4)_2H]^+ B(3,5-bis(trifluormethyl)-phenyl)_4^-$,

$[Cp*Rh(P(OMe)_3)(CH_2=CHCO_2Me)(Me)]^+ B(3,5-bis(trifluormethyl)-phenyl)_4^-$,

$[Cp*Rh(-CH_2-CH_2CO_2Me)(P(OMe)_3)]^+ B(3,5-bis(trifluormethyl)-phenyl)_4^-$,

$[Cp*Rh(MeO_2C(CH_2)_2-(CH-)-(CH_2)CO_2Me)]^+ B(3,5-bis(trifluormethyl)-phenyl)_4^-$,

$[Cp*Rh(C_2H_4)_2H]^+ B(perfluorphenyl)_4^-$,

$[Cp*Rh(P(OMe)_3)(CH_2=CHCO_2Me)(Me)]^+ B(perfluorphenyl)_4^-$,

$[Cp*Rh(-CH_2-CH_2CO_2Me)(P(OMe)_3)]^+ B(perfluorphenyl)_4^-$ und

$[Cp*Rh(MeO_2C(CH_2)_2-(CH-)-(CH_2)CO_2Me)]^+ B(perfluorphenyl)_4^-$

$[Cp*Rh(C_2H_4)_2H]^+Al(OR^F)_4^-$,

$[Cp*Rh(P(OMe)_3)(CH_2=CHCO_2Me)(Me)]^+ Al(OR^F)_4^-$,

$[Cp*Rh(-CH_2-CH_2CO_2Me)(P(OMe)_3)]^+Al(OR^F)_4^-$ und

$$[Cp*Rh(MeO_2C(CH_2)_2\text{-}(CH\text{-})\text{-}(CH_2)CO_2Me)]^+ Al(ORF)_4^-,$$

wobei $R^F$ für gleiche oder unterschiedliche teilfluorierte oder perfluorierte aliphatische oder aromatische Reste, insbesondere für Perfluor-iso-propyl oder Perfluor-tert.-butyl steht.

Solche Katalysatoren und ihre Herstellung kann nach an sich bekannten Verfahren erfolgen, wie sie beispielsweise in EP-A-475 386, JACS 1991, 113, 2777-2779, JACS 1994, 116, 8038-8060 beschrieben sind.

Die Hydrierung kann derart durchgeführt werden, daß die monoolefinisch ungesättigte Verbindung, die mindestens zwei funktionelle Gruppen, unabhängig voneinander ausgewählt aus der Gruppe bestehend aus Nitrilgruppe, Carbonsäuregruppe, Carbonsäureestergruppe, Carbonsäureamidgruppe, trägt, zu einer gesättigten Verbindung unter Erhalt der genannten funktionellen Gruppen umgesetzt wird. Diese Hydrierung kann vorteilhaft bei einem Wasserstoff-Partialdruck im Bereich von 0,01 bis 20 MPa durchgeführt werden. Bei der Hydrierung hat sich eine durchschnittliche mittlere Verweilzeit der monoolefinisch ungesättigten Verbindung, die mindestens zwei funktionelle Gruppen, unabhängig voneinander ausgewählt aus der Gruppe bestehend aus Nitrilgruppe, Carbonsäuregruppe, Carbonsäureestergruppe, Carbonsäureamidgruppe, trägt, im Bereich von 0,1 bis 100 Stunden als vorteilhaft erwiesen. Weiterhin kommt für die Hydrierung vorzugsweise eine Temperatur im Bereich von 30°C bis 160°C in Betracht.

Die Hydrierung kann derart durchgeführt werden, daß die monoolefinisch ungesättigten Verbindung, die mindestens zwei funktionelle Gruppen, unabhängig voneinander ausgewählt aus der Gruppe bestehend aus Nitrilgruppe, Carbonsäuregruppe, Carbonsäureestergruppe, Carbonsäureamidgruppe, trägt, zu einer gesättigten Verbindung unter Hydrierung mindestens einer, vorzugsweise aller der genannten funktionellen Gruppen, besonders bevorzugt einer oder mehrerer Gruppen, ausgewählt aus Carbonsäuregruppe und Carbonsäureestergruppe, insbesondere Carbonsäureestergruppe, umgesetzt wird, insbesondere unter Umwandlung der genannten Gruppe oder Gruppen in eine oder mehrere Gruppen der Struktur -CH$_2$OH. Diese Hydrierung kann vorteilhaft bei einem Wasserstoff-Partialdruck im Bereich von 10 bis 30 MPa durchgeführt werden. Bei der Hydrierung hat sich eine durchschnittliche mittlere Verweilzeit der monoolefinisch ungesättigten Verbindung, die mindestens zwei funktionelle Gruppen, unabhängig voneinander ausgewählt aus der Gruppe bestehend aus Nitrilgruppe, Carbonsäuregruppe, Carbonsäureestergruppe, Carbonsäureamidgruppe, trägt, im Bereich von 0,1 bis 100 Stunden als vorteilhaft erwiesen. Weiterhin kommt für die Hydrierung vorzugsweise eine Temperatur im Bereich von 200°C bis 350°C in Betracht.

Die Vorteile der Hydrierung zwischen den Schritten a) und b) kommen besonders zum Tragen, wenn man mindestens 0,5 %, vorzugsweise midnestens 1 %, insbesondere mindestens 5 % der eingesetzten monoolefinisch ungesättigten Verbindung, die mindestens zwei funktionelle Gruppen, unabhängig voneinander ausgewählt aus der Gruppe bestehend aus Nitrilgruppe, Carbonsäuregruppe, Carbonsäureestergruppe, Carbonsäureamidgruppe, trägt, zu einer gesättigten Verbindung, die die gleichen mindestens zwei funktionellen Gruppen trägt, hydriert.

Erfindungsgemäß führt man die in Schritt a) erhaltene Mischung, gegebenenfalls nach einer Hydrierung zwischen den Schritten a) und b), einem Schritt b) zu, in dem man die Mischung destilliert unter Erhalt

b1) der durch einfache Addition der genannten zwei terminalen Olefine erhaltenen Verbindung, die mindestens zwei funktionelle Gruppen, unabhängig voneinander ausgewählt aus der Gruppe bestehend aus Nitrilgruppe, Carbonsäuregruppe, Carbonsäureestergruppe, Carbonsäureamidgruppe trägt, als Kopfprodukt und

b2) einer Mischung enthaltend

b2a) durch einfache Addition der genannten zwei terminalen Olefine erhaltene Verbindung, die mindestens zwei funktionelle Gruppen, unabhängig voneinander ausgewählt aus der Gruppe bestehend aus Nitrilgruppe, Carbonsäuregruppe, Carbonsäureestergruppe, Carbonsäureamidgruppe trägt,

b2b) eine durch mehrfache Addition der genannten zwei terminalen Olefine erhaltene Verbindung und

b2c) die als Katalysator für diese Addition geeignete, bezüglich der Reaktionsmischung homogene Verbindung.

Die Destillation gemäß Schritt b) kann man vorteilhaft bei einer Sumpftemperatur im Bereich von 50 bis 200°C, vorzugsweise 60 bis 160°C, insbesondere 70 bis 150°C durchführen.

Hierbei kommen Drücke, gemessen im Sumpf der Destillationsvorrichtung, im Bereich von 0,05 bis 50 kPa, vorzugsweise 0,1 bis 10 kPa, insbesondere 0,2 bis 6 kPa in Betracht.

Dabei haben sich durchschnittliche mittlere Verweilzeiten im Bereich von 1 bis 45 Minuten, vorzugsweise 5 bis 35 Minuten, insbesondere 10 bis 25 Minuten als vorteilhaft erwiesen.

Für die Destillation kommen hierfür übliche Apparaturen in Betracht, wie sie beispielsweise in: Kirk-Othmer, Encyclopedia of Chemical Technology, 3.Ed., Vol. 7, John Wiley & Sons, New York, 1979, Seite 870-881 beschrieben sind, wie Siebbodenkolonnen, Glockenbodenkolonnen, Packungskolonnen, Füllkörperkolonnen, Dualflowbodenkolonnen, Ventilbodenkolonnen oder einstufige Verdampfer, wie Fallfilmverdampfer, Dünnschichtverdampfer oder Flashverdampfer.

Die Destillation kann man in mehreren, wie 2 oder 3 Apparaturen, vorteilhaft einer einzigen Apparatur durchführen.

Die als Kopfprodukt b1) erhaltene Komponente kann, wenn gewünscht, nach an sich bekannten Verfahren aufgearbeitet oder weiterverarbeitet werden.

Wurde als Kopfprodukt eine ungesättigte Verbindung erhalten, so kann diese nach an sich bekannten Verfahren zu einer gesättigten Verbindung hydriert werden. So kann beispielsweise eine ungesättigte Dicarbonsäure oder deren Ester, wie Diester, beispielsweise Butendicarbonsäure oder deren Mono- oder Diester, zu der entsprechenden gesättigten Dicarbonsäure oder deren Ester, wie Diester, beispielsweise Adipinsäure oder deren Mono- oder Diester, oder zu dem entsprechenden, insbesondere gesättigten Alkohol, beispielsweise Hexan-1,6-diol, umgesetzt werden.

Wurde als Kopfprodukt b1) ein Diester, wie Adipinsäurediester oder Butendicarbonsäurediester, erhalten, so kann dieser beispielsweise vorteilhaft mit einer terminal ungesättigten Carbonsäure, wie Acrylsäure, umgesetzt werden unter Erhalt einer Dicarbonsäure, wie Butendicarbonsäure oder Adipinsäure, und dem entsprechenden Ester der terminal ungesättigten Carbonsäure. Solche Verfahren sind beispielsweise in der deutschen Anmeldung 10240781.9 beschrieben.

Erfindungsgemäß erfolgt in Schritt c) eine Auftrennung der in Schritt b2) erhaltenen Mischung mittels einer semipermeablen Membran unter Erhalt eines Permeats und eines Retentats, derart, daß das Gewichts-Verhältnis der Komponente b2b) zur Komponente b2c) in der Schritt c) zugeführten Mischung b2) kleiner ist als im Retentat.

Als semipermeable Membranen kommen vorzugsweise solche in Betracht, die für Komponente b2c) eine höhere Durchlässigkeit aufweisen als für Komponente b2b).

Weiterhin kommen als semipermeable Membranen vorzugsweise solche in Betracht, die für Komponente b2a) eine höhere Durchlässigkeit aufweisen als für Komponente b2b).

Eine Trennschicht der semipermeablen Membranen kann eines oder mehrere organische oder anorganische Materialien enthalten, insbesondere ausgewählt aus der Gruppe bestehend aus organisches Polymer, keramische Materialien, Metalle und Kohlenstoff oder deren Kombinationen. Sie sollten bei der Filtrationstemperatur im Feedmedium stabil sein.

Als Keramik kommen vorzugsweise alpha-Aluminiumoxid, Zirkoniumoxid, Titandioxid, Siliziumcarbid oder gemischte keramische Werkstoffe in Betracht.

Als organisches Polymer kann man vorteilhaft Polypropylen, Polytetrafluorethylen, Polyvinylidendifluorid, Polysulfon, Polyethersulfon, Polyetherketon, Polyamid, Polyimid, Polyacrylnitril, Regeneratcellulose oder Silikon einsetzen.

Aus mechanischen Gründen sind die Trennschichten in der Regel auf einer ein- oder mehrschichtigen porösen Unterstruktur aus dem gleichen oder einem anderen Material wie die Trennschicht aufgebracht. Die Unterschicht ist im allgemeinen grobporiger als die Trennschicht. Beispiele für vorteilhafte Materialkombinationen sind in der folgenden Tabelle aufgeführt:

| Trennschicht | Unterschicht |
|---|---|
| Metall | Metall |
| Keramik | Metall, Keramik oder Kohlenstoff |
| Polymer | Polymer, Metall, Keramik oder Keramik auf Metall |
| Kohlenstoff | Kohlenstoff, Metall oder Keramik |

Die mittlere durchschnittliche Porengröße der Membran sollte vorteilhaft im Bereich von 0,9 bis 50 nm, insbesondere 3 bis 20 nm im Falle von anorganischen Membranen betragen. Die Trenngrenzen sollten bevorzugt im Bereich von 500 bis 100000 Dalton, insbesondere im Bereich von 2000 bis 40000 Dalton im Falle von organischen Membranen liegen.

Die Membranen können in verschiedenen Geometrien, wie Flach-, Rohr-, Multikanalelement-, Kapillar- oder Wickelgeometrie, eingesetzt werden, für die entsprechende Druckgehäuse, die eine Trennung zwischen Retentat und Permeat erlauben, verfügbar sind.

Die optimalen transmembranen Drücke sind im wesentlichen abhängig vom Durchmesser der Membranporen, den hydrodynamischen Bedingungen, die de Deckschichtaufbau beeinflussen, und der mechanischen Stabilität der Membran bei der Filtrationstemperatur.

In einer bevorzugten Ausführungsform kann der transmembrane Druck im Bereich von 0,02 bis 10 MPa, insbesondere 0,1 bis 6 MPa betragen.

Das Verhältnis des Drucks auf der Retentatseite zu dem Druck auf der Permearseite der Membran kann vorzugsweise im Bereich von 2 bis 100 liegen.

Auf der Retentatseite kann man vorteilhaft einen Druck im Bereich von 0,1 bis 10 MPa anwenden.

Auf der Permeatseite kann man vorteilhaft einen Druck im Bereich von 1 bis 1000 kPa anwenden.

Die Membrantrennung kann insbesondere bei einer Temperatur im Bereich von 0 bis 150°C durchgeführt werden.

Um einen nennenswerten Aufbau aus einer Deckschicht aus Komponente b) zu vermeiden, der zu einer deutlichen Abnahme des Permeatflusses führt, haben sich Umpumpen, mechanische Bewegung der Membran oder Rühraggregate zwischen den Membranen als nützlich erwiesen, insbesondere zur Erzeugung einer Relativgeschwindigkeit zwischen Membran und Suspension im Bereich von 0,1 bis 10 m/s.

Die Permeatflüsse sollten vorteilhaft im Bereich von 1 bis 50 kg/m$^2$/h liegen.

Die Membrantrennung kann kontinuierlich erfolgen, beispielsweise durch einmaligen Durchgang durch eine oder mehrere nacheinandergeschaltete Membran-Trennstufen.

Die Membrantrennung kann diskontinuierlich erfolgen, beispielsweise durch mehrmaligen Durchgang durch die Membranmodule.

Bei der Membrantrennung können Hilfsstoffe eingesetzt werden. Hierbei hat sich bevorzugt der Einsatz von Komponente a) oder b1) als vorteilhaft erwiesen, insbesondere in dem Umfang, in dem Komponente a) oder b1) als Permeat abgezogen wird.

Aus dem Retentat kann dann Komponente a) oder b1) durch an sich bekannte Verfahren, beispielsweise durch Destillation, Extraktion, Membrantrennung, vorzugsweise durch Destillation, abgetrennt werden.

Hierzu kommen die bereits für Schritt b) beschriebenen Parameter und Apparaturen in Betracht.

In einer bevorzugten Ausführungsform kann das in dem erfindungsgemäßen Verfahren erhaltene Permeat teilweise oder vollständig in Schritt a) zurückgeführt werden.

In einer weiteren bevorzugten Ausführungsform kann die in der Destillation erfindungsgemäß erhaltene Mischung b2) ganz oder teilweise der erfindungsgemäßen Membrantrennung zugeführt werden. Der demgemäß anfallende Teilstrom, der nicht der erfindungsgemäßen Membrantrennung zugeführt wird, kann teilweise oder ganz, bevorzugt ganz, in Schritt a) zurückgeführt werden.

Beispiele

Definitionen

Transmembrandruck:

$$TMP = ((P_{Moduleingang} + P_{Modulausgang})/2) - P_{Permeat}$$

Losungsmittelaustauschkoeffizient bei der Diafiltration:

MA = Diafiltriermittelzugabe (kg)/ Anlageninhalt (kg)

Beispiel 1

Dimerisierung eines funktionalisierten Olefins, die destillative Abtrennung des homogenen Katalysators und die Abtrennung von Hochsiedern durch Membrantrennung

Ein gerührter Glasautoklav mit einem Innenvolumen von 750 mL und ein gerührter Glasautoklav mit einem Innenvolumen von 400 mL sind als Reaktoren R1 bzw. R2 in Reihe geschaltet. Mit Hilfe einer Pumpe P1 wird dem ersten Autoklaven MA als Edukt zugeführt. Die Zuführung erfolgt über ein Tauchrohr in den Flüssigkeitsraum des R1. Ebenfalls über diese Leitung wird Wasserstoff gasförmig über einen Massendurchflussregler F1 eingeleitet. Der Stand des R1 wird über ein zweites Tauchrohr eingestellt, das als Überlauf zu R2 dient. In die Überlaufleitung zum R2 wird ebenfalls gasförmiger Wasserstoff über einen Massendurchflussregler F2 dosiert. Der Zulauf zu R2 wird ebenfalls über ein Tauchrohr in R2 eingetragen und über ein weiteres Tauchrohr der Austrag aus R2 über ein Druckregulierventil der Fa. Reco in einen Dünnschichtverdampfer mit einer Verdampferfläche von 0,046 m$^2$ geführt. Der Verdampfer wird über eine Vakuumstation auf einen vorgegebenen Druck eingestellt. Der Verdampfer wird mit einem Ölbad W1 beheizt. Über die Temperatur in W1 wird der Stand im Ablaufgefäß des Dünnschichtverdampfers geregelt. Aus diesem Gefäß fördert eine Pumpe P2 einen Kreislaufstrom über den Verdampfer und eine weitere Pumpe P3 aus diesem Kreislauf einen Rückführungsstrom in den Reaktor R1, der ebenfalls über das Tauchrohr eingeleitet wird, über das auch der MA-Zulauf dosiert wird. Die Pumpen P1 und P3 fördern jeweils die gleichen Volumina pro Zeit. Der Brüdenstrom des Verdampfers wird über einen Intensivkühler geführt und dort kondensiert. Das Kondensat wird anschlieβend gesammelt (Austrag). Die unter diesen Bedingungen nicht kondensierten Bestandteile werden einer Kondensation bei Normaldruck unterworfen und in einer Kühlfalle gesammelt.

Betrieb der kontinuierlichen Dimerisierung und Katalysatorabtrennung:

Zu Versuchsbeginn werden die Reaktoren mit einer Lösung gefüllt, die $Cp*Rh(C_2H_4)_2$ und eine stöchiometrische Menge $HBArF_4$ sowie 250 ppm PTZ in HDME enthält. Zur Erreichung einer gleichmäßigen Durchmischung wird der Reaktionsansatz zunächst bei Raumtemperatur für ca. 20 h im Kreis gefahren. Danach wird der Dünnschichtverdampfer auf eine Starttemperatur von 100°C vorgeheizt. Dann werden der Wasserstoffstrom und der MA-Zulauf (120 ml/h, enthält 100 Gew.-ppm PTZ) gestartet, die Reaktoren auf 70°C geheizt und der Verdampfer wird im Vakuum betrieben.
Im stabilen Zustand wird für den R1 eine Rhodium-Konzentration von 190 ppm bestimmt. In einem repräsentativen Bilanzzeitraum von 18 h werden folgende Ergebnisse erhalten:

| | |
|---|---|
| Feed: | 2264 g |
| Kühlfalle: | 222 g (81 % MA) |
| Austrag: | 2036 g (95 % ungesättigte lineare Diester, 4 % MA, ca. 0.5 % DMA) |

Nach einer Reihe von Bilanzen steigt der Anteil von Hochsiedern im Katalysatorkreislauf. Daher wird ein Teil des Rückführstromes ausgeschleust und mit MA auf ein Gesamtgewicht von 3002,6 g verdünnt. Die Zusammensetzung dieser Lösung ist wie folgt gekennzeichnet:

| | |
|---|---|
| Rh: | 16 ppm |
| Hochsieder: | 65 g/kg (Rückstandsbestimmung: Verdampfung im Vak. bei 250°C) |

Die Lösung wird einer kontinuierlichen Membranfiltration unterworfen, die in Beispiel 4 näher beschrieben wird.
Das MA und Rhodium-Katalysator enthaltende Permeat aus Beispiel 4 konnte direkt als Feed in der kontinuierlichen Anlage zur Dimerisierung eingesetzt werden und somit eine Rückführung des Katalysators bei gleichzeitiger Abtrennung des Polymers erreicht werden.

Beispiel 2

Dimerisierung eines funktionalisierten Olefins mit der Hydrierung der C-C-Doppelbindung des Produktes mit einem Rhodium-haltigen Katalysator sowie destillative Abtrennung des homogenen Katalysators und die Abtrennung von Hochsiedern durch Membrantrennung
Es wird eine Laborapparatur wie in Beispiel 1 beschrieben verwendet. Lediglich wird der Feed nicht in R1 dosiert, sondern in R2.
Zu Versuchsbeginn werden die Reaktoren mit einer Lösung gefüllt, die $Cp*Rh(C_2H_4)_2$ und eine stöchiometrische Menge $HBArF_4$ sowie 250 ppm PTZ in HDME enthält. Zur Erreichung einer gleichmäßigen Durchmischung wird der Reaktionsansatz zunächst bei Raumtemperatur für ca. 20 h im Kreis gefahren. Danach wird der Dünnschichtverdampfer auf eine Starttemperatur von 100°C vorgeheizt. Dann werden der Wasserstoffstrom und der MA-Zulauf (120 ml/h, enthält 100 Gew.-ppm PTZ) gestartet, die Reaktoren auf 70°C geheizt und der Verdampfer wird im Vakuum betrieben. Der Wasserstoff in diesem Beispiel enthält 50 ppm $O_2$.
Nach mehreren Tagen ist ein stabiler Zustand erreicht. In einem repräsentativen Bilanzzeitraum von 18 h werden folgende Ergebnisse erhalten.

| | |
|---|---|
| Rh-Konz. R1: | 175 ppm |
| Rh-Konz. R2: | 110 ppm |
| Feed: | 725 g |
| Kühlfalle: | 383 g (99 % MA) |
| Austrag: | 284 g (63 % ungesättigte lineare Diester, 20 % DMA, 17 % MA) |

Das gebildete Polymer kann wie in den Beispielen 3-5 beschrieben abgetrennt werden.

Beispiele 3-5 (Membranfiltration)

Abtrennung des homogen gelösten Rhodium-Katalysators von hochsiedenden Verbindungen

Für die Versuche wurde eine thermostatisierbare Kreislaufapparatur mit einem minimalen hold up von 3 l eingesetzt.

In dem Kreislauf waren ein Vorratsbehälter, eine Pumpe zur Druckerzeugung und Überströmung der Membran, ein Wärmetauscher zur Haltung der Temperatur, ein Membranmodul mit eingebauter keramischer Rohrmembran und ein Druckhalteventil integriert. Der Permeatablauf war drucklos. Über eine Standhaltung konnte der Hold up der Anlage konstantgehalten werden (Diafiltrationsmodus). Alle Vorlagen der Apparatur wurde mit Stickstoff inertisiert. Die eingesetzte keramische Rohrmembran (Hersteller: Inocermic GmbH) hatte einen Außendurchmesser von 10 mm, einen Innendurchmesser von 7 mm und eine Länge von 1000 mm. Der Stützkörper bestand aus $Al_2O_3$ und die innen aufgebrachte Trennschicht enthielt 5 nm Poren aus $TiO_2$. Die Membran wurde von innen angeströmt und das Permeat auf der Außenseite abgeführt.

Allgemeine Versuchsdurchführung

3 kg Destillationssumpf wurden in den Kreislaufbehälter eingetragen, dann bei geschlossenem Permeatabgang die Pumpe gestartet und der Druck vor der Membran, die Überströmung sowie die Temperatur auf Sollwert gebracht. Die Temperatur betrug 40°C und die Überströmung 4 m/s im Membranrohr. Dann wurde der Permeat-abgang geöffnet und die Nachführung des Diafiltriermediums aktiviert. Nach einer gewissen Permeatabnahme und der gleich großen Zufuhr von Diafiltriermedium wurde der Versuch abgebrochen. Dann wurden der Retentateinsatz, Retentataustrag und das Mischpermeat in Bezug auf den Hochsieder (Polymer) und Katalysator analysiert.

Die nachfolgende Tabelle enthält die Ergebnisse von kontinuierlich betriebenen Membranfiltrationen deren Parameter zuvor beschrieben wurden. Das Beispiel 4 beschreibt die Membranfiltration eines Teilstromes aus Beispiel 1.

Tabelle 1:

| Ergebnisse der Membranfiltrationen | | | | | | | | | | | | | | |
|---|---|---|---|---|---|---|---|---|---|---|---|---|---|---|
| Beispiel Nr. | TMP | Permeatfluss | Retentateinsatz | | | | Retentataustrag | | | | Diafiltriermedium | MA | Permeataustrag | | |
| | (bar) | (kg/m²/h) | m (kg) (kg) | Polymer (%) | Rh (ppm) | Rh/Polymer (ppm%) | m (kg) (kg) | Polymer (%) | Rh (ppm) | Rh/Polymer (ppm/%) | | | m (kg) (kg) | Polymer (%) | Rh (ppm) |
| 3 | 5 | 13 | 3,2 | 1,0 | 360 | 360 | 3,2 | 1 | 55 | 55 | HDME | 3,4 | 10,8 | n.n | 92 |
| 4 | 5 | 15 | 3,6 | 6,5 | 16,0 | 2,46 | 3,6 | 6,5 | 3,5 | 0,54 | Methylacrylat | 2,5 | 9,1 | n.n | 5,0 |
| 5 | 1 | 18 | 3,0 | 4,8 | 100 | 20,8 | 3,0 | 4,8 | 85 | 17,7 | Aceton | 2,7 | 8,3 | n.n | 5,5 |
| n.n. = nicht nachweisbar | | | | | | | | | | | | | | | |

**Patentansprüche**

1. Verfahren zur kontinuierlichen Herstellung einer Verbindung, die mindestens zwei funktionelle Gruppen, unabhängig voneinander ausgewählt aus der Gruppe bestehend aus Nitrilgruppe, Carbonsäuregruppe, Carbonsäureestergruppe, Carbonsäureamidgruppe, trägt, umfassend die Schritte

   a) Addition zweier terminaler Olefine, die die zur Herstellung der mindestens zwei funktionellen Gruppen enthaltenden Verbindung gemäß a1) erforderlichen funktionellen Gruppen tragen, in Gegenwart eines als Katalysator für diese Addition geeigneten, bezüglich der Reaktionsmischung homogenen Verbindung gemäß a3) unter Erhalt einer Mischung, enthaltend

   a1) eine durch einfache Addition der genannten zwei terminalen Olefine erhaltene Verbindung, die mindestens zwei funktionelle Gruppen, unabhängig voneinander ausgewählt aus der Gruppe bestehend aus Nitrilgruppe, Carbonsäuregruppe, Carbonsäureestergruppe, Carbonsäureamidgruppe trägt,
   a2) eine durch mehrfache Addition der genannten zwei terminalen Olefine erhaltene Verbindung und
   a3) die als Katalysator für diese Addition geeignete, bezüglich der Reaktionsmischung homogene Verbindung

   b) Destillation der in Schritt a) erhaltenen Mischung unter Erhalt

   b1) der durch einfache Addition der genannten zwei terminalen Olefine erhaltenen Verbindung, die mindestens zwei funktionelle Gruppen, unabhängig voneinander ausgewählt aus der Gruppe bestehend aus Nitrilgruppe, Carbonsäuregruppe, Carbonsäureestergruppe, Carbonsäureamidgruppe trägt, als Kopfprodukt und
   b2) einer Mischung enthaltend
   b2a) durch einfache Addition der genannten zwei terminalen Olefine erhaltene Verbindung, die mindestens zwei funktionelle Gruppen, unabhängig voneinander ausgewählt aus der Gruppe bestehend aus Nitrilgruppe, Carbonsäuregruppe, Carbonsäureestergruppe, Carbonsäureamidgruppe trägt,
   b2b) eine durch mehrfache Addition der genannten zwei terminalen Olefine erhaltene Verbindung und
   b2c) die als Katalysator für diese Addition geeignete, bezüglich der Reaktionsmischung homogene Verbindung,

   c) Auftrennung der gesamten in Schritt b2) erhaltenen Mischung oder eines Teils davon mittels einer semipermeablen Membran unter Erhalt eines Permeats und eines Retentats derart, daß das Gewichts-Verhältnis der Komponente b2b) zur Komponente b2c) in der Schritt c) zugeführten Mischung b2) kleiner ist als im Retentat,
   d) das in Schritt c) erhaltene Permeat teilweise oder vollständig in Schritt a) zurückführt
   und
   e) den nicht gemäß c) aufgetrennten Teil der in Schritt b2) erhaltenen Mischung teilweise oder vollständig in Schritt a) zurückführt,

   wobei man als terminale Olefine zwei Olefine einsetzt, die unabhängig voneinander die Formel $H_2C=CHR^1$ aufweisen, in der $R^1$ für eine Nitrilgruppe, Carbonsäuregruppe, Carbonsäureestergruppe, Carbonsäureamidgruppe, steht.

2. Verfahren nach Anspruch 1, wobei man zwischen den Schritten a) und b) die Verbindung gemäß a1) in der in Schritt a) erhaltenen Mischung hydriert unter Erhalt einer gesättigten Verbindung.

3. Verfahren nach Anspruch 1 oder 2, wobei man die Addition gemäß Schritt a) in Gegenwart einer bezüglich der Reaktionsmischung homogenen Verbindung, die Rhodium, Ruthenium, Palladium oder Nickel enthält, als Katalysator durchführt.

4. Verfahren nach den Ansprüchen 1 oder 2, wobei man die Addition gemäß Schritt a) in Gegenwart einer bezüglich der Reaktionsmischung homogenen, Rhodium enthaltenden Verbindung als Katalysator durchführt.

5. Verfahren nach den Ansprüchen 2 bis 4, wobei man die Hydrierung zwischen den Schritten a) und b) in Gegenwart einer bezüglich der Reaktionsmischung homogenen Verbindung, die Rhodium, Ruthenium, Palladium oder Nickel enthält, als Katalysator durchführt.

6. Verfahren nach den Ansprüchen 2 bis 4, wobei man die Hydrierung zwischen den Schritten a) und b) in Gegenwart einer bezüglich der Reaktionsmischung homogenen Verbindung, die Rhodium enthält, als Katalysator durchführt.

7. Verfahren nach den Ansprüchen 2 bis 6, wobei man bei der Addition gemäß Schritt a) und der Hydrierung zwischen den Schritten a) und b) die gleiche Verbindung als Katalysator einsetzt.

8. Verfahren nach den Ansprüchen 1 bis 7, wobei man die Addition gemäß Schritt a) oder die Hydrierung oder beides in Gegenwart einer bezüglich der Reaktionsmischung homogenen, Rhodium enthaltenden Verbindung der Formel $[L^1RHL^2L^3R]^+X^-$ als Katalysator durchführt, worin

$L^1$ ein anionischer Pentahapto-Ligand ist;
$L^2$ für einen neutralen 2-Elektronendonor steht;
$L^3$ für einen neutralen 2-Elektronendonor steht;
R ausgewählt wird aus der Gruppe, bestehend aus H, $C_1$-$C_{10}$-Alkyl-, $C_6$-$C_{10}$-Aryl- und $C_7$-$C_{10}$-Aralkyl-Liganden
$X^-$ für ein nichtkoordinierendes Anion steht;

und worin zwei oder drei von $L^2$, $L^3$ und R gegebenenfalls verbunden sind.

9. Verfahren nach Anspruch 8, worin $L^1$ Pentamethylcyclopentadienyl ist.

10. Verfahren nach den Ansprüchen 8 und 9, worin X- ausgewählt ist aus der Gruppe bestehend aus $BF_4^-$, B(perfluor-phenyl)$_4^-$, B(3,5-bis(trifluormethyl)-phenyl)$_4^-$, Al(OR$^F$)$_4^-$ wobei R$^F$ für gleiche oder unterschiedliche teilfluorierte oder perfluorierte aliphatische oder aromatische Reste steht.

11. Verfahren nach Anspruch 8 bis 10, wobei $L^2$ und $L^3$ unabhängig voneinander ausgewählt sind aus der Gruppe bestehend aus $C_2H_4$, $CH_2=CHCO_2Me$, $P(OMe)_3$ und $MeO_2C$-$(C_4H_6)$-$CO_2Me$.

12. Verfahren nach den Ansprüchen 8 bis 10, wobei $L^2$ und $L^3$ zusammen ausgewählt sind aus der Gruppe bestehend aus Acrylnitril und 5-Cyanopentensäureester.

13. Verfahren nach den Ansprüchen 8 bis 11, wobei $L^2$ und R zusammen -$CH_2$-$CH_2CO_2Me$ darstellen.

14. Verfahren nach den Ansprüchen 8 bis 11 oder 13, wobei $L^2$, $L^3$ und R zusammen $MeO_2C(CH_2)_2$-(CH)-$(CH_2)CO_2Me$ darstellen.

15. Verfahren nach Anspruch 8, wobei man die Addition gemäß Schritt a) oder die Hydrierung oder beides durchführt in Gegenwart einer bezüglich der Reaktionsmischung homogenen, Rhodium enthaltenden Verbindung als Katalysator, ausgewählt aus der Gruppe bestehend aus

$[Cp^*Rh(C_2H_4)_2H]^+BF_4^-$,

$[Cp^*Rh(P(OMe)_3)(CH_2=CHCO_2Me)(Me)]^+ BF_4^-$,

$[Cp^*Rh(-CH_2-CH_2CO_2Me)(P(OMe)_3)^+ BF_4^-$,

$[Cp^*Rh(MeO_2C(CH_2)_2-(CH-)-(CH_2)CO_2Me)]^+ BF_4^-$,

$[Cp^*Rh(C_2H_4)_2H]^+ B(3,5-bis(trifluormethyl)-phenyl)_4^-$,

$[Cp^*Rh(P(OMe)_3)(CH_2=CHCO_2Me)(Me)]^+ B(3,5-bis(trifluormethyl)-phenyl)_4^-$,

$[Cp^*Rh(-CH_2-CH_2CO_2Me)(P(OMe)_3)]^+ B(3,5-bis(trifluormethyl)-phenyl)_4^-$,

$[Cp^*Rh(MeO_2C(CH_2)_2-(CH-)-(CH_2)CO_2Me)^+ B(3,5-bis(trifluormethyl)-phenyl)_4^-$,

$[Cp^*Rh(C_2H_4)_2H]^+ B(perfluorphenyl)_4^-$,

$[Cp^*Rh(P(OMe)_3)(CH_2=CHCO_2Me)(Me)]^+B(perfluorphenyl)_4^-$,

$[Cp^*Rh(-CH_2-CH_2CO_2Me)(P(OMe)_3)]^+$ $B(perfluorphenyl)_4^-$ $[Cp^*Rh(MeO_2C(CH_2)_2$-

$(CH-)-(CH_2)CO_2Me)]^+B(perfluorphenyl)_4^-$

$[Cp^*Rh(C_2H_4)_2H]^+Al(OR^F)_4^-$,

$[Cp^*Rh(P(OMe)_3)(CH_2=CHCO_2Me)(Me)]^+ Al(OR^F)4^-$,

$[Cp^*Rh(-CH_2-CH_2CO_2Me)(P(OMe)_3)]^+ Al(OR^F)_4^-$ und

$[Cp^*Rh(MeO_2C(CH_2)_2-(CH-)-(CH_2)CO_2Me)]^+ Al(OR^F)_4^-$,

wobei $R^F$ für gleiche oder unterschiedliche teilfluorierte oder perfluorierte aliphatische oder aromatische Reste steht.

16. Verfahren nach den Ansprüchen 1 bis 15, wobei man die Hydrierung bei einem Wasserstoff-Partialdruck im Bereich von 10 bis 20000 kPa durchführt.

17. Verfahren nach den Ansprüchen 1 bis 16, wobei man die Hydrierung bei einer durchschnittlichen mittleren Verweilzeit der monoolefinisch ungesättigten Verbindung, die mindestens zwei funktionelle Gruppen, unabhängig voneinander ausgewählt aus der Gruppe bestehend aus Nitrilgruppe, Carbonsäuregruppe, Carbonsäureestergruppe, Carbonsäureamidgruppe, trägt, im Bereich von 0,1 bis 100 Stunden beträgt.

18. Verfahren nach den Ansprüchen 2 bis 17, wobei man die in der Addition gemäß Schritt a) erhaltene Mischung ohne Abtrennung der als Katalysator eingesetzten, Rhodium enthaltenden Verbindung einer Hydrierung gemäß den Ansprüchen 1 bis 17 zuführt.

19. Verfahren nach den Ansprüchen 1 bis 18, wobei man die Destillation gemäß Schritt b) bei einer Temperatur im Bereich von 50 bis 200°C durchführt.

20. Verfahren nach den Ansprüchen 1 bis 19, wobei man die Destillation gemäß Schritt b) bei einer durchschnittlichen mittleren Verweilzeit im Bereich von 1 bis 45 Minuten durchführt.

21. Verfahren nach den Ansprüchen 1 bis 20, wobei man die Destillation gemäß Schritt b) bei einem Druck im Bereich von 0,5 bis 500 mbar durchführt.

22. Verfahren nach den Ansprüchen 1 bis 21 wobei man eine Membran enthaltend im wesentlichen ein oder mehrere organische oder anorganische Materialien.

23. Verfahren nach den Ansprüchen 1 bis 22, wobei die mittlere durchschnittliche Porengröße der Membran im Bereich von 0,9 bis 50 nm im Falle von anorganischen Membranen beträgt.

24. Verfahren nach den Ansprüchen 1 bis 22, wobei die mittlere durchschnittliche Trenngrenze der Membran im Bereich von 500 bis 100000 Dalton im Falle von organischen Membranen beträgt.

25. Verfahren nach den Ansprüchen 1 bis 24, wobei das Verhältnis des Drucks auf der Retentatseite der Membran zu dem Druck auf der Permeatseite der Membran im Bereich von 2 bis 100 liegt.

26. Verfahren nach den Ansprüchen 1 bis 25, wobei man auf der Retentatseite der Membran einen Druck im Bereich von 0,1 bis 10 MPa anwendet.

27. Verfahren nach den Ansprüchen 1 bis 26, wobei man auf der Permeatseite der Membran einen Druck im Bereich von 1 bis 1000 kPa anwendet.

28. Verfahren nach den Ansprüchen 1 bis 27, wobei man die Membrantrennung bei einer Temperatur im Bereich von 0 bis 150°C durchführt.

29. Verfahren nach den Ansprüchen 1 bis 28, wobei man die in Schritt b) als b1) erhaltene Komponente hydriert unter Erhalt einer gesättigten Verbindung.

**Claims**

1. A process for continuously preparing a compound which bears at least two functional groups which are each independently selected from the group consisting of nitrile group, carboxylic acid group, carboxylic ester group and carboxamide group, comprising the steps of

    a) adding two terminal olefins which bear the functional groups required to prepare the compound as per a1) comprising at least two functional groups, in the presence of a compound as per a3) which is suitable as a catalyst for this addition and is homogeneous with respect to the reaction mixture to obtain a mixture comprising

    a1) a compound which is obtained by monoaddition of the two terminal olefins mentioned and bears at least two functional groups which are each independently selected from the group consisting of nitrile group, carboxylic acid group, carboxylic ester group and carboxamide group,
    a2) a compound which is obtained by polyaddition of the two terminal olefins mentioned and
    a3) the compound which is suitable as a catalyst for this addition and is homogeneous with respect to the reaction mixture,

    b) distilling the mixture obtained in step a) to obtain

    b1) the compound which is obtained by monoaddition of the two terminal olefins mentioned and bears at least two functional groups which are each independently selected from the group consisting of nitrile group, carboxylic acid group, carboxylic ester group and carboxamide group, as the top product and
    b2) a mixture comprising
    b2a) a compound which is obtained by monoaddition of the two terminal olefins mentioned and bears at least two functional groups which are each independently selected from the group consisting of nitrile group, carboxylic acid group, carboxylic ester group and carboxamide group,
    b2b) a compound which is obtained by polyaddition of the two terminal olefins mentioned and
    b2c) the compound which is suitable as a catalyst for this addition and is homogeneous with respect to the reaction mixture,

    c) separating the entire mixture obtained in step b2) or a portion thereof by means of a semipermeable membrane to obtain a permeate and a retentate, in such a way that the weight ratio of component b2b) to component b2c) in the mixture b2) fed in step c) is smaller than in the retentate,
    d) recycling the permeate obtained in step c) partly or fully into step a)
    and
    e) recycling the portion of the mixture obtained in step b2) which has not been separated in c) partly or fully into step a),

    wherein the terminal olefins used are two olefins which each independently have the formula $H_2C=CHR^1$ in which $R^1$ is a nitrile group, carboxylic acid group, carboxylic ester group or carboxamide group.

2. The process according to claim 1, wherein the compound as per a1) in the mixture obtained in step a) is hydrogenated between steps a) and b) to obtain a saturated compound.

3. The process according to claim 1 or 2, wherein the addition in step a) is carried out in the presence of a compound, as a catalyst, which is homogeneous with respect to the reaction mixture and comprises rhodium, ruthenium, palladium or nickel.

4. The process according to either of claims 1 and 2, wherein the addition in step a) is carried out in the presence of a compound, as a catalyst, which is homogeneous with respect to the reaction mixture and comprises rhodium.

5. The process according to any of claims 2 to 4, wherein the hydrogenation between steps a) and b) is carried out in the presence of a compound, as a catalyst, which is homogeneous with respect to the reaction mixture and comprises rhodium, ruthenium, palladium or nickel.

6. The process according to any of claims 2 to 4, wherein the hydrogenation between steps a) and b) is carried out in the presence of a compound, as a catalyst, which is homogeneous with respect to the reaction mixture and comprises rhodium.

7. The process according to any of claims 2 to 6, wherein the same compound is used as a catalyst in the addition in step a) and the hydrogenation between steps a) and b).

8. The process according to any of claims 1 to 7, wherein the addition in step a) or the hydrogenation or both are carried out in the presence of a compound, as a catalyst, which is homogeneous with respect to the reaction mixture, comprises rhodium and has the formula $[L^1RhL^2L^3R]^+X^-$ where

$L^1$ is an anionic pentahapto ligand;
$L^2$ is an uncharged 2-electron donor;
$L^3$ is an uncharged 2-electron donor;
R is selected from the group consisting of H, $C_1$-$C_{10}$-alkyl, $C_6$-$C_{10}$-aryl and $C_7$-$C_{10}$-aralkyl ligands
$X^-$ is a noncoordinating anion;

and where two or three of $L^2$, $L^3$ and R are optionally joined.

9. The process according to claim 8, wherein $L^1$ is pentamethylcyclopentadienyl.

10. The process according to either of claims 8 and 9, wherein $X^-$ is selected from the group consisting of $BF_4^-$, B(perfluorophenyl)$_4^-$, B(3,5-bis(trifluoromethyl)phenyl)$_4^-$, Al(ORF)$_4^-$ where $R^F$ is identical or different part-fluorinated or perfluorinated aliphatic or aromatic radicals.

11. The process according to any of claims 8 to 10, wherein $L^2$ and $L^3$ are each independently selected from the group consisting of $C_2H_4$, $CH_2$=$CHCO_2Me$, P(OMe)$_3$ and MeO$_2$C-($C_4H_6$)-CO$_2$Me.

12. The process according to any of claims 8 to 10, wherein $L^2$ and $L^3$ together are selected from the group consisting of acrylonitrile and 5-cyanopentenoic ester.

13. The process according to any of claims 8 to 11, wherein $L^2$ and R together are -CH$_2$-CH$_2$CO$_2$Me.

14. The process according to any of claims 8 to 11 or 13, wherein $L^2$, $L^3$ and R together are MeO$_2$C(CH$_2$)$_2$-(CH)-(CH$_2$)CO$_2$Me.

15. The process according to claim 8, wherein the addition in step a) or the hydrogenation or both are carried out in the presence of a compound, as a catalyst, which is homogeneous with respect to the reaction mixture, contains rhodium and is selected from the group consisting of

[Cp*Rh(C$_2$H$_4$)$_2$H]$^+$ BF$_4^-$,

[Cp*Rh(P(OMe)$_3$)(CH$_2$=CHCO$_2$Me)(Me)]$^+$BF$_4^-$,

[Cp*Rh(-CH$_2$-CH$_2$CO$_2$Me)(P(OMe)$_3$)]$^+$BF$_4^-$,

[Cp*Rh(MeO$_2$C(CH$_2$)$_2$-(CH-)-(CH$_2$)CO$_2$Me)]$^+$BF$_4^-$,

[Cp*Rh(C$_2$H$_4$)$_2$H]$^+$B(3,5-bis(trifluoromethyl)phenyl)$_4^-$,

[Cp*Rh(P(OMe)$_3$)(CH$_2$=CHCO$_2$Me)(Me)]$^+$B(3,5-bis(trifluoromethyl)phenyl)$_4^-$,

[Cp*Rh(-CH$_2$-CH$_2$CO$_2$Me)(P(OMe)$_3$)$^+$B(3,5-bis(trifluoromethyl)phenyl)$_4^-$,

[Cp*Rh(MeO$_2$C(CH$_2$)$_2$-(CH-)-(CH$_2$)CO$_2$Me)]$^+$B(3,5-bis(trifluoromethyl)phenyl)$_4^-$,

[Cp*Rh(C$_2$H$_4$)$_2$H]$^+$B(perfluorophenyl)$_4^-$,

[Cp*Rh(P(OMe)$_3$)(CH$_2$=CHCO$_2$Me)(Me)]$^+$ B(perfluorophenyl)$_4^-$,

[Cp*Rh(-CH$_2$-CH$_2$CO$_2$Me)(P(OMe)$_3$)]$^+$B(perfluorophenyl)$_4^-$,

[Cp*Rh(MeO$_2$C(CH$_2$)$_2$-(CH-)-(CH$_2$)CO$_2$Me)]$^+$ B(perfluorophenyl)$_4$$^-$,

[Cp*Rh(C$_2$H$_4$)$_2$H]$^+$Al(OR$^F$)$_4$$^-$,

[Cp*Rh(P(OM$_e$)$_3$)(CH$_2$=CHCO$_2$Me)(Me)]$^+$Al(OR$^F$)$_4$$^-$,

[Cp*Rh (-CH$_2$-CH$_2$CO$_2$Me) (P (OMe)$_3$)] Al (OR$^F$)$_4$$^-$ and

[Cp*Rh (MeO$_2$C (CH$_2$) $_2$- (CH-) - (CH$_2$) CO$_2$Me)]$^+$ Al (OR$^F$)$_4$$^-$,

where R$^F$ is identical or different part-fluorinated or perfluorinated aliphatic or aromatic radicals.

16. The process according to any of claims 1 to 15, wherein the hydrogenation is carried out at a partial hydrogen pressure in the range from 10 to 20 000 kPa.

17. The process according to any of claims 1 to 16, wherein the hydrogenation is at an average mean residence time of the monoolefinically unsaturated compound which bears at least two functional groups which are each independently selected from the group consisting of nitrile group, carboxylic acid group, carboxylic ester group and carboxamide group in the range from 0.1 to 100 hours.

18. The process according to any of claims 2 to 17, wherein the mixture obtained in the addition in step a) is fed to a hydrogenation according to any of claims 1 to 18 without removing the rhodium-comprising compound used as a catalyst.

19. The process according to any of claims 1 to 18, wherein the distillation in step b) is carried out at a temperature in the range from 50 to 200°C.

20. The process according to any of claims 1 to 19, wherein the distillation in step b) is carried out at an average mean residence time in the range from 1 to 45 minutes.

21. The process according to any of claims 1 to 20, wherein the distillation in step b) is carried out at a pressure in the range from 0.5 to 500 mbar.

22. The process according to any of claims 1 to 21, wherein a membrane which comprises substantially one or more organic or inorganic materials.

23. The process according to any of claims 1 to 22, wherein the mean average pore size of the membrane is in the range from 0.9 to 50 nm in the case of inorganic membranes.

24. The process according to any of claims 1 to 22, wherein the mean average separation limit of the membrane is in the range from 500 to 100 000 daltons in the case of organic membranes.

25. The process according to any of claims 1 to 24, wherein the ratio of the pressure on the retentate side of the membrane to the pressure on the permeate side of the membrane is in the range from 2 to 100.

26. The process according to any of claims 1 to 25, wherein a pressure in the range from 0.1 to 10 MPa is applied on the retentate side of the membrane.

27. The process according to any of claims 1 to 26, wherein a pressure in the range from 1 to 1 000 kPa is applied on the permeate side of the membrane.

28. The process according to any of claims 1 to 27, wherein the membrane separation is carried out at a temperature in the range from 0 to 150°C.

29. The process according to any of claims 1 to 28, wherein the component obtained as b1) in step b) is hydrogenated to obtain a saturated compound.

**Revendications**

1. Procédé pour la préparation continue d'un composé qui porte au moins deux groupes fonctionnels, choisis indépendamment l'un de l'autre dans le groupe constitué par le groupe nitrile, le groupe acide carboxylique, un groupe ester d'acide carboxylique, un groupe amide d'acide carboxylique, comprenant les étapes

   a) addition de deux oléfines terminales, qui portent les groupes fonctionnels nécessaires pour la préparation dudit composé contenant au moins deux groupes fonctionnels selon a1), en présence d'un composé selon a3), approprié comme catalyseur pour cette addition, homogène par rapport au mélange réactionnel avec obtention d'un mélange, contenant

   a1) un composé obtenu par simple addition des deux oléfines terminales mentionnées, qui porte au moins deux groupes fonctionnels, choisis indépendamment l'un de l'autre dans le groupe constitué par le groupe nitrile, le groupe acide carboxylique, un groupe ester d'acide carboxylique, un groupe amide d'acide carboxylique,
   a2) un composé obtenu par addition multiple des deux oléfines terminales mentionnées et
   a3) le composé approprié comme catalyseur pour cette addition, homogène par rapport au mélange réactionnel

   b) distillation du mélange obtenu dans l'étape a) avec obtention

   b1) du composé obtenu par simple addition des deux oléfines terminales mentionnées, qui porte au moins deux groupes fonctionnels, choisis indépendamment l'un de l'autre dans le groupe constitué par le groupe nitrile, le groupe acide carboxylique, un groupe ester d'acide carboxylique, un groupe amide d'acide carboxylique, comme produit de tête et
   b2) d'un mélange contenant
   b2a) un composé obtenu par simple addition des deux oléfines terminales mentionnées, qui porte au moins deux groupes fonctionnels, choisis indépendamment l'un de l'autre dans le groupe constitué par le groupe nitrile, le groupe acide carboxylique, un groupe ester d'acide carboxylique, un groupe amide d'acide carboxylique,
   b2b) un composé obtenu par addition multiple des deux oléfines terminales mentionnées et
   b2c) le composé approprié comme catalyseur pour cette addition, homogène par rapport au mélange réactionnel,

   c) séparation du mélange total obtenu dans l'étape b2) ou d'une partie de celui-ci au moyen d'une membrane semi-perméable avec obtention d'un perméat et d'un retentat, de manière telle que le rapport pondéral du composant b2b) au composant b2c) dans le mélange b2) introduit dans l'étape c) est inférieur à celui dans le retentat,
   d) recyclage partiel ou complet du perméat obtenu dans l'étape c) dans l'étape a) et
   e) recyclage partiel ou complet de la partie non séparée selon c) du mélange obtenu dans l'étape b2) dans l'étape a),

   en utilisant, comme oléfines terminales, deux oléfines qui présentent, indépendamment l'une de l'autre, la formule $H_2C=CHR^1$, dans laquelle $R^1$ représente un groupe nitrile, le groupe acide carboxylique, le groupe ester d'acide carboxylique, un groupe amide d'acide carboxylique.

2. Procédé selon la revendication 1, le composé selon a1) dans le mélange obtenu dans l'étape a) étant hydrogéné, entre les étapes a) et b), avec obtention d'un composé saturé.

3. Procédé selon la revendication 1 ou 2, l'addition selon l'étape a) étant réalisée en présence d'un composé homogène par rapport au mélange réactionnel, qui contient du rhodium, du ruthénium, du palladium ou du nickel, comme catalyseur.

4. Procédé selon les revendications 1 ou 2, l'addition selon l'étape a) étant réalisée en présence d'un composé homogène par rapport au mélange réactionnel, qui contient du rhodium, comme catalyseur.

5. Procédé selon les revendications 2 à 4, l'hydrogénation entre les étapes a) et b) étant réalisée en présence d'un composé homogène par rapport au mélange réactionnel, qui contient du rhodium, du ruthénium, du palladium ou du nickel, comme catalyseur.

**6.** Procédé selon les revendications 2 à 4, l'hydrogénation entre les étapes a) et b) étant réalisée en présence d'un composé homogène par rapport au mélange réactionnel, qui contient du rhodium, comme catalyseur.

**7.** Procédé selon les revendications 2 à 6, l'addition selon l'étape a) et l'hydrogénation entre les étapes a) et b) étant réalisées en présence du même composé comme catalyseur.

**8.** Procédé selon les revendications 1 à 7, l'addition selon l'étape a) ou l'hydrogénation ou les deux étant réalisée(s) en présence d'un composé homogène par rapport au mélange réactionnel, contenant du rhodium, de formule $[L^1RhL^2L^3R]^+X^-$ comme catalyseur, dans laquelle

$L^1$ représente un ligand pentahapto anionique ;
$L^2$ représente un donneur de 2 électrons, neutre ;
$L^3$ représente un donneur de 2 électrons, neutre ;
R est choisi dans le groupe constitué par H, les ligands $C_1$-$C_{10}$-alkyle, $C_6$-$C_{10}$-aryle et $C_7$-$C_{10}$-aralkyle ;
$X^-$ représente un anion non coordinant ;

et dans laquelle deux ou trois parmi $L^2$, $L^3$ et R sont le cas échéant reliés.

**9.** Procédé selon la revendication 8, $L^1$ représentant pentaméthylcyclopentadiényle.

**10.** Procédé selon les revendications 8 et 9, X étant choisi dans le groupe constitué par $BF_4^-$, B(perfluorophényle)$_4^-$, B(3,5-bis(trifluorométhyl)-phényle)$_4^-$, Al $(OR^F)_4$, $R^F$ représentant des radicaux aliphatiques ou aromatiques, partiellement fluorés ou perfluorés, identiques ou différents.

**11.** Procédé selon la revendication 8 à 10, $L^2$ et $L^3$ étant choisis, indépendamment l'un de l'autre, dans le groupe constitué par $C_2H_4$, $CH_2$=$CHCO_2Me$, $P(OMe)_3$ et $MeO_2C$-$(C_4H_6)$-$CO_2Me$.

**12.** Procédé selon les revendications 8 à 10, $L^2$ et $L^3$ étant choisis, ensemble, dans le groupe constitué par l'acrylonitrile et les esters de l'acide 5-cyanopenténoïque.

**13.** Procédé selon les revendications 8 à 11, $L^2$ et R représentant, ensemble, -$CH_2$-$CH_2CO_2Me$.

**14.** Procédé selon les revendications 8 à 11 ou 13, $L^2$, $L^3$ et R représentant, ensemble, $MeO_2C(CH_2)_2$-(CH)-$(CH_2)CO_2Me$.

**15.** Procédé selon la revendication 8, l'addition selon l'étape a) ou l'hydrogénation ou les deux étant réalisée(s) en présence d'un composé homogène par rapport au mélange réactionnel, contenant du rhodium, comme catalyseur, choisi dans le groupe constitué par

$[Cp^*Rh(C_2H_4)_2H]^+BF_4^-$,

$[Cp^*Rh(P(OMe)_3)(CH_2=CHCO_2Me)(Me)]^+BF_4^-$,

$[Cp^*Rh(-CH_2-CH_2CO_2Me)(P(OMe)_3)]^+BF_4^-$,

$[Cp^*Rh(MeO_2C(CH_2)_2-(CH-)-(CH_2)CO_2Me)]^+BF_4^-$,

$[Cp^*Rh(C_2H_4)_2H]^+B(3,5-bis(trifluorométhyl)-phényle)_4^-$,

$[Cp^*Rh (P (OMe)_3) (CH_2=CHCO_2Me) (Me)]^+ B(3,5-bis(trifluorométhyl)-phényle)_4^-$,

$[Cp^*Rh (-CH_2-CH_2CO_2Me) (P (OMe)_3) ]^+ B(3,5-bis(trifluorométhyl)-phényle)_4^-$,

$[Cp^*Rh (MeO_2C (CH_2)_2- (CH-) - (CH_2) CO_2Me)]^+B (3, 5-bis(trifluorométhyl)-phényle)_4^-$,

$[Cp^*Rh(C_2H_4)_2H]^+B(perfluorophényle)_4^-$,

$[Cp*Rh(P(OM_e)_3)(CH_2=CHCO_2Me)\ (Me)]^+\ B(perfluorophényle)_4^-,$

$[Cp*Rh\ (-CH_2-CH_2CO_2Me)\ (P(OMe)_3)]^+\ B\ (perfluorophényle)_4^-$

$[Cp*Rh\ (MeO_2C\ (CH_2)_2-\ (CH-)\ -\ (CH_2)\ CO_2Me)]^+$

$B(perfluorophényle)_4^-$

$[Cp*Rh(C_2H_4)_2H]^+\ Al(OR^F)_4^-,$

$[Cp*Rh(P(OM_e)_3)\ (CH_2=CHCO_2Me)\ (Me)]^+\ Al(ORF)_4^-,$

$[Cp*Rh(-CH_2-CH_2CO_2Me)\ (P\ (OMe)_3)]^+Al\ (OR^F)_4^-$ et

$[Cp*Rh\ (MeO_2C\ (CH_2)_2-\ (CH-)\ -\ (CH_2)CO_2Me)]^+\ Al\ (OR^F)_4^-,$

$R^F$ représentant des radicaux aliphatiques ou aromatiques, partiellement fluorés ou perfluorés, identiques ou différents.

16. Procédé selon les revendications 1 à 15, l'hydrogénation étant réalisée à une pression partielle d'hydrogène dans la plage de 10 à 20 000 kPa.

17. Procédé selon les revendications 1 à 16, l'hydrogénation étant à un temps de séjour moyen du composé oléfiniquement monoinsaturé, qui porte au moins deux groupes fonctionnels, choisis indépendamment l'un de l'autre dans le groupe constitué par le groupe nitrile, le groupe acide carboxylique, un groupe ester d'acide carboxylique, un groupe amide d'acide carboxylique, dans la plage de 0,1 à 100 heures.

18. Procédé selon les revendications 2 à 7, le mélange obtenu dans l'addition selon l'étape a) étant introduit sans séparation du composé utilisé comme catalyseur, contenant du rhodium, dans une hydrogénation selon les revendications 1 à 17.

19. Procédé selon les revendications 1 à 18, la distillation selon l'étape b) étant réalisée à une température dans la plage de 50 à 200°C.

20. Procédé selon les revendications 1 à 19, la distillation selon l'étape b) étant réalisée à un temps de séjour moyen dans la plage de 1 à 45 minutes.

21. Procédé selon l'une quelconque des revendications 1 à 20, la distillation selon l'étape b) étant réalisée à une pression dans la plage de 0,5 à 500 mbars.

22. Procédé selon les revendications 1 à 21, une membrane contenant essentiellement un ou plusieurs matériaux organiques ou inorganiques.

23. Procédé selon les revendications 1 à 22, la grosseur moyenne des pores de la membrane se situant dans la plage de 0,9 à 50 nm dans le cas de membranes inorganiques.

24. Procédé selon les revendications 1 à 22, la limite de séparation moyenne de la membrane se situant dans la plage de 500 à 100 000 daltons dans le cas de membranes organiques.

25. Procédé selon les revendications 1 à 24, le rapport de la pression côté retentat de la membrane à la pression côté perméat de la membrane se situant dans la plage de 2 à 100.

26. Procédé selon les revendications 1 à 25, une pression dans la plage de 0,1 à 10 MPa étant utilisée côté retentat de la membrane.

27. Procédé selon les revendications 1 à 26, une pression dans la plage de 1 à 1000 kPa étant utilisée côté perméat de la membrane.

28. Procédé selon les revendications 1 à 27, la séparation membranaire étant réalisée à une température dans la plage de 0 à 150°C.

29. Procédé selon les revendications 1 à 28, le composant obtenu dans l'étape b) en tant que b1) étant hydrogéné avec obtention d'un composé saturé.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4451665 A
- FR 2524341
- US 4889949 A
- US 4594447 A
- US 3013066 A
- US 4638084 A
- EP 475386 A
- DE 10240781

**In der Beschreibung aufgeführte Nicht-Patentliteratur**

- *J. Organomet. Chem.,* 1987, vol. 320, C56
- *Organometallics,* 1986, vol. 5, 1752
- *J. Mol. Catal.,* 1993, vol. 85, 149
- *Angew. Chem. Int. Ed. Engl.,* 1988, vol. 27, 185
- *JACS,* 1991, vol. 113, 2777-2779
- *JACS,* 1994, vol. 116, 8038-8060
- **KIRK-OTHMER.** Encyclopedia of Chemical Technology. John Wiley & Sons, 1979, vol. 7, 870-881